# EUROPEAN PATENT APPLICATION

(11) **EP 2 272 980 A1**
(43) Date of publication of application: **12.01.2011**
(21) Application number: 09425276.4
(22) Date of filing: 09.07.2009
(51) Int. Cl.: C12Q 1/68

(54) **Molecular probes for target nucleic acid detection and quantification**

(71) Applicant: Bioesplora SRL, 72018 San Michele Salentino (BR) (IT)
(72) Inventor: D'Urso, Massimiliano, 72018 San Michele Salentino (BR) (IT); D'Urso, Oscar Fernando, 72018 San Michele Salentino (BR) (IT); D'Urso, Pietro Ivo, 72018 San Michele Salentino (BR) (IT)

(57) **Abstract**

The present invention described and claimed herein relate to the design and construction of nucleic acid probes to Staphylococcus aureus which are capable of detecting the organism in test samples of, e.g., sputum, urine, blood and tissue sections, food, soil and water. The present invention design the overall probe composed of specific functional subsequences comprising the target recognition site, two universal primer sequence, two locking sequences and a 5' flanking sequence. This probe design allow the probe amplification, by means of a universal primer pair, only in the presence of the target sequence. Multiple probes can be used simultaneously which can be distinguished by means of length differences (PCR) or by means of the binding of another labelled probe (real-time quantitative PCR). This method is characteristic of fast, easily operating, high sensitivity, high specificity, and the interpretation of the result accurate and reliable. It's suitable for large scale screen in both clinical or agriculture applications.

## Description

### Technical Field

The present invention relates to the field of genetic engineering, particularly, relates to a probe useful for detecting a specific target sequence in a complex matrix and in particular for the detection of Staphylococcus specific target DNA/RNA sequences. More particularly, relates to real time quantitative TaqMan or to end-point PCR probe useful for detecting and quantifying a target sequence. The present invention further relates to the use of the probe and related product in the preparation of kit or identical product useful for detecting and quantify one or more target sequences in a complex matrix.

### Background of the invention

Two single strands of deoxyribo- ("DNA") or ribo- ("RNA") nucleic acid, formed from nucleotides (including the bases adenine (A), cytosine (C), thymidine (T), guanine (G), uracil (U), or inosine (I)), may associate ("hybridize") to form a double stranded structure in which the two strands are held together by hydrogen bonds between pairs of complementary bases. Generally, A is hydrogen bonded to T or U, while G is hydrogen bonded to C.

When a first single strand of nucleic acid contains sufficient contiguous complementary bases to a second, double stranded nucleic acid will result. Under appropriate conditions, DNA/DNA, RNA/DNA, or RNA/RNA hybrids may be formed. A probe is generally a single stranded nucleic acid sequence which is complementary to some degree to a nucleic acid sequence sought to be detected ("target sequence"). It may be labelled with a detectable moiety such as a radioisotope, antigen or chemiluminescent moiety.

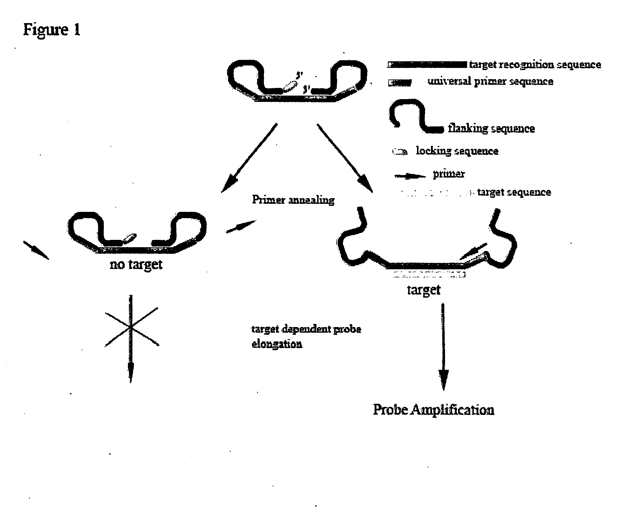

The high complexity of mammalian genomes requires preamplification of DNA targets before analysis. Most detection techniques use PCR as a necessary first step for complexity reduction. Multiplex PCR (mpxPCR) provides simultaneous amplification of many targets of interest in one reaction, thus increasing the assay throughput and allowing more efficient use of each DNA sample. Most multiplex reactions are restricted to amplification of few targets. The reason for this is that with each primer set added, the reaction conditions allowing efficient amplification of each DNA fragment become increasingly less flexible. Several attempts have been made to overcome some of the difficulties with mpxPCR. One of the suggested methods is based on an additional step yielding a long DNA fragment, which is then used as a template for reamplification of short amplicons. This method, however, does not offer a general solution for amplification of multiple targets from different parts of complex genomes. Another improvement to mpxPCR uses two rounds of amplification. The first round is performed with a relatively low concentration of gene-specific primers containing a universal or zip code sequence, whereas the second round uses a high concentration of shorter primers corresponding to the zip code. Nevertheless, the maximal achievable multiplexing by using conventional PCR remains restricted to about 10 targets and requires tedious optimization of PCR conditions. Here we describe a new procedure for increasing the PCR multiplexing level. We use the PCR suppression (PS) effect. This allows PCR amplification with only two primers common for all targets and correspond to an adapter ligated to both ends of all probes (**Fig. 1**). In PS-based PCR (PS PCR) the adapter is about 40 bases long and has a high GC content. During PCR, after each denaturation step, self-complementary GC-rich ends of single-stranded (ss) fragments form strong duplexes; thus, all ss DNA fragments adopt hairpin structures. Replication of such DNA fragments using the primer corresponding to the adapter (A primer) is suppressed. However, synthesis can occur from a primer **(T primer)** complementary to a universal primer sequence located within the ss loop of the hairpin structure, and this product is then amplified efficiently by the **A and T primers.** As a result, efficient PCR is possible only for target bound probes. Because the **A and T** primers are the same for all probes, such PCR allows amplification with only one universal primer pair. PS PCR has been used successfully in a variety of applications: genome walking, cDNA subtractive hybridization, and targeted genomic differential display. Here we show that PS PCR also allows efficient multiplex target amplification with allele specificity.

### Summary of the invention

Staphylococcus aureus is the causative agent of a wide variety of infections in humans, including diseases of the skin and soft tissues. e.g., skin pustules, impetigo, bacteremia, osteomyelitis, renal abscess, pneumonia, meningitis, endocarditis, gastroenteritis and toxic shock syndrome (Kloos and Schleifer, Staphylococcus, In Bergey's Manual of Systematic Bacteriology, 1013-1019, 1986). Further, S. aureus is a prominent agent of nosocomial infections. Current methods used to identify Staphylococcus aureus include Gram Stain morphology, cell morphology, production of catalase, pigment production, susceptibility to lysostaphin and lysozyme and anaerobic production of acid from glucose (Kloos and Schleifer, Simplified Scheme for Routine Identification of Human Staphylococcus Species, 1J. Clin. Microbiol. 82, 1975). In addition, there are several commercially available systems that allow strains to be biochemically characterized.

Among Staphylococcus species associated with human infections, S. aureus is unique in its ability to clot plasma. It should be noted, however, that some Staphylococcus species found in animals, such as S. intermedius and S. hyicus may also share this property. Two different coagulase tests are commonly used to identify S. aureus. One is a tube test for free coagulase, and the other is a slide test for bound coagulase. The tube coagulase test is thought be be the more definitive of the two, however it can take several hours, or even an overnight incubation period to produce a result. Using the slide coagulase test, up to 10 to 15 percent of S. aureus stains may yield a negative result (Jorgeuen and Kloos, Staphylococcal Infections, Diagnostic Procedures for Bacterial Infections, 7th ed. B. B. Wentworth. American Public Health Association, Washington, DC 1987). Thus, in a first aspect, the invention features an assay probe able to distinguish Staphylococcus aureus from other Staphylococcus species. In preferred embodiments, the probe is complementary to rRNA or rDNA, e.g., a variable region of rRNA; at least 50% of the nucleotides in the target recognition sequence of the probe are able to hybridize to a contiguous series of bases in at least one variable region of ribosomal nucleic acid in Staphylococcus aureus; the target recognition sequence of the probe is a nucleotide polymer able to hybridize to the rRNA of the species Staphylococcus aureus in the region corresponding to bases 338-367 of E. coli 23S rRNA, or a nucleotide polymer complementary thereto.

By "consists essentially of" is meant that the probe is provided as a purified nucleic acid which hybridizes under stringent hybridizing conditions with the desired organism and not with other related organisms. Such a probe may be linked to other nucleic acids which do not affect such hybridization. Generally, it is preferred that the probe be of between 15 and 100 (most preferably between 20 and 50) bases in size. It may, however, be provided in a vector.

In related aspects, the invention features a nucleotide polymer able to hybridize to the above oligonucleotides, a nucleic acid hybrid formed with the above oligonucleotides, and a nucleic acid sequence substantially complementary thereto.

The probes of this invention offer a rapid, non-subjective method of identification and quantitation of a bacterial colony for the presence of specific rRNA sequences unique to all strains of Staphylococcus aureus.

Other features and advantages of the invention will be apparent from the following description of the preferred embodiments thereof, and from the claims.

The objects of the invention was achieved through the following principles: In order to detect the presence of a target sequence, a probe was designed which including the sequence complementary to the target sequence, two universal primer sequences, two locking sequences and a 5' flanking sequence.

When the probe is free in solution (not bound to the target) the two locking sequences bind to complementary sequences presents on the universal primer sequences and in the target region creating a stem and loop structure which prevents the binding of the primers to the universal primer sequences. So in this circumstances no amplification of the probe is allowed. In addition when the probe is not bound to its target the 5' flanking sequence prevents unspecific probe amplification due to 5'-3' polimerization of the self-hybridized probe. On the other side when the probe bind a target sequence the stem and loop structure is prevented and so the universal primer sequences can bind the primers and the amplification process take place. This method is able to detect nucleic acid target in fewer than femptogram.

Some modification have to be introduced in a standard PCR reaction. In fact after the denaturation step at 95°C follow two annealing step: one at an higher temperature that will allow the binding of the target recognition sequence of the probe to the target sequence or the self-annealing of the flanking sequences on the probe (This last will happen only in the case the target sequence is not present). The second annealing step will allow the binding of the primers to the universal primer sequences only in the case the probe is bound to the target. After the two annealing steps it will follows the polymerization step in which a polymerase enzyme catalyze the polymerization of the bound primers using as template the same probe. This process is a target mediated probe amplification. In fact the target presence allows to the probe to obtain an open structure which in turn allows the binding of the primers to the universal primer sequences which will be used by a polymerase to amplify the probe.

In another embodiment, after the denaturation step of the PCR reaction one or more annealing steps allow the hybridization of the primers to the universal primer sequences only for the probes which are hybridized to the target sequence resulting in this way with a target mediated probe amplification.

Multiple probes can be used simultaneously in the same reaction allowing the detection of several targets. The revelation process can be obtained into the following ways:
1. End-point PCR
2. Real-time PCR
3. microarray

### End-point PCR

To obtain the revelation of several probes simultaneously it is mandatory to select the target sequence length and the universal primer sequence length so that the sum of these lengths are different.

### Real-time PCR

To reveal the presence of the amplified probe in real-time PCR it will be used specific target sequence probes labelled with specific fluorophores like taq-man probes.

Since the product of amplification is proportional to the starting target concentration this approach can be used also for quantification of the target sequence.

### Microarray

To reveal the presence of the amplified probe it can be contructed an array composed of oligos complementary to target recognition sequence allowing in this way the simultaneous detection of multiple amplification reactions in a single step

This approach can be used for the detection of bacteria in clinical, environmental and food matrix. In fact this approach is able to detect quantities lower than femptograms. The presence of a bacteria in a matrix is always coupled with the presence of its DNA or RNA. For this reason if the probe is developed based, for example, on a specific sequence of the *Listeria monocytogenes,* or *Salmonella spp, E. coli, Staphylococcus spp, Streptococcus spp.* etc. DNA sequence we will be able to detect the presence of this bacteria in the starting matrix. In addition if information on the viability of the bacteria wants to be obtained it will be developed probes on specific RNA species.

This probes can be used also in the gene expression profiling, in fact it will be sufficient to design a target recognition sequence to be specific for a desired target RNA sequence and this will allow the quantification of the target itself.

Although it is desirable to detect the presence of nucleic acids as described above, often the sought nucleic acid sequences are present in sample sources in extremely small numbers (e. g., less than 110). The condition of small target molecule numbers causes a requirement that laboratory techniques be performed in order to amplify the numbers of the target sequences in order that they may be detected. There are many well known methods of amplifying targeted sequences, such as the polymerase chain reaction (PCR), the ligase chain reaction (LCR), the strand displacement amplification (SDA), and the nucleic acid sequence-based amplification (NASBA), to name a few. These methods are described generally in the following references: (PCR) U. S. Patent Nos. 4,683,195,4,683,202, and 4,800,159; (LCR) EP Application No., 320,308 published June 14,1989; (SDA) U. S. Patent Nos. 5,270,184, and 5,455,166 and"Empirical Aspects of Strand Displacement Amplification"by G. T. Our method is able to detect target nucleic acid sequences in also this small numbers since is based on the amplification of the probe and not on the target.

With respect to analyzing and/or identifying target nucleic acid amplified products, i. e., other well known techniques have been typically used including hybridization analysis, comparative size, etc. However, these methods, are neither rapid nor sensitive as compared to the teachings of the present invention.

In real time quantitative PCR amplification system, a forward primer and a reverse primer (useful for amplifying simultaneously all the probes in the mixture thanks to the same universal primer sequences) allow the amplification of the probes which are bound to the specific target sequence. In association a second specific real-time probe (like Taqman probe) can be used to detect and quantify the desired probes and so to indirectly to detect and quantify the target sequence.

Therefore, based upon the inventive concepts, in one aspect, the present invention provides a target mediated probe amplification method able to detect and quantify simultaneously several target DNA or RNA (after a reverse transcription step) sequénces.

### Figure Legends

In the Figure 1 is reported a drawing showing the principle of functioning of the probe.

### EXAMPLES

### EXAMPLE 1

To detect a quantify the presence of Staphylococcus aureus in a test sample. In this example we report the case in which a preliminary nucleic acid purification is preferred. When the solution containing the nucleic acid derived from test sample is ready one mixes an aliquot of this solution with the reaction mix containing a polymerase with all the required reagents, the probe object of the invention and the universal primer. When the mixture is introduced in a termal cycler the correct and sequencially use of denaturation step, one or more annealing steps and the elongation step allow the amplification of the probe only if the starting test sample contained the Staphylococcus DNA.

### EXAMPLE 2

In another embodiment no preliminary nucleic acid purification step is required. When the test sample is ready one mixes an aliquot of this solution with the reaction mix containing a polymerase with all the required reagents, the probe object of the invention and the universal primer. When the mixture is introduced in a termal cycler the correct and sequencially use of denaturation step, one or more annealing steps and the elongation step allow the amplification of the probe only if the starting test sample contained the Staphylococcus DNA.

### Bibliographic page

1. Chamberlain, J. S., Gibbs, R. A., Ranier, J. E., Nguyen, P. N. & Caskey, C. T. (1988) Nucleic Acids Res. 16, 11141-11156.
2. Edwards, M. C. &. Gibbs R. A. (1994) PCR Methods Appl. 3, S65-S75.
3. Hacia, J. G., Sun, B., Hunt, N., Edgemon, K., Mosbrook, D., Robbins, C., Fodor, S. P., Tagle, D. A. & Collins F. S. (1998) Genome Res. 8, 1245-1258.
4. Li, D. & Vijg, J. (1996) Nucleic Acids Res. 24, 538-539.
5. Stuven, T., Griese, E. U., Kroemer, H. K., Eichelbaum, M. & Zanger, U. M. (1996) Pharmacogenetics 6, 417-421.
6. van Orsouw, N. J., Zhang, X., Wei, J. Y., Johns, D. R. & Vijg, J. (1998) Genomics 52, 27-36.
7. Shuber, A. P., Grondin, V. J. & Klinger, K. W. (1995) Genome Res. 5, 488-493.
8. Belgrader, P., Marino, M. M., Lubin, M. & Barany, F. (1996) Genome Science & Technology 1, 77-87.
9. Launer, G. A., Lukyanov, K. A., Tarabykin, V. S. &. Lukyanov, S. A. (1994) Mol. Genet. Mikrobiol. Virusol. 6, 38-41.
10. Siebert, P. D., Chenchik, A., Kellogg, D. E., Luckyanov, K. A. & Lukyanov, S. A. (1995) Nucleic Acids Res. 23, 1087-1088.

## Claims

1. The present invention relates to a molecular probe useful for detecting and quantify the presence of a specific target nucleic acid sequence in a complex matrix. The present invention design the overall probe composed of specific functional subsequences comprising the target recognition site, two universal primer sequence, two locking sequences and a 5' flanking sequence. This probe design allow the probe amplification, by means of a universal primer pair, only in the presence of the target sequence. Multiple probes can be used simultaneously which can be distinguished by means of length differences (PCR) or by means of the binding of another labelled probe (real-time quantitative PCR). This method is characteristic of fast, easily operating, high sensitivity, high specificity, and the interpretation of the result accurate and reliable. It's suitable for large scale screen in both clinical or agriculture applications.

2. A molecular probe able to detect Staphylococcus aureus derived nucleic acids.

3. A kit for Staphylococcus detection in food or environmental matrixes

4. A kit for Staphylococcus detection in clinical sample
